# EUROPÄISCHE PATENTSCHRIFT

(11) **EP 0 508 326 B1**
(45) Veröffentlichungstag und Bekanntmachung des Hinweises auf die Patenterteilung: **27.08.1997**
(21) Anmeldenummer: 92105809.5
(22) Anmeldetag: 03.04.1992
(51) Int. Cl.: A61N 1/39

(54) **Implantierbarer Defibrillator**
Implantable defibrillator
Défibrillateur implantable

(30) Priorität: 09.04.1991 DE 4111478
(43) Veröffentlichungstag der Anmeldung: 14.10.1992
(73) Patentinhaber: Pacesetter AB, 171 95 Solna (SE)
(72) Erfinder: Andersen, Hans, S-162 35 Vällingby (SE); Obel, Martin, S-182 33 Danderyd (SE); Wallén, Lars, S-172 34 Sundbyberg (SE)
(74) Vertreter: Lettström, Richard Wilhelm

(56) Entgegenhaltungen:
- DE-A- 1 927 667
- DE-A- 2 811 325
- DE-A- 2 938 708
- US-A- 4 300 567

## Beschreibung

Die Erfindung betrifft einen implantierbaren Defibrillator mit einer steuerbaren Schalteranordnung (3), mit Elektrodenanschlüssen (5,6), mit einem Kondensator, der über eine steuerbare Schalteranordnung wechselweise mit einer Spannungsquelle oder die Elektrodenansclüsse (5,6) verbindbar ist, und mit einer die Schalteranordnung steuernden Steuereinrichtung.

Bei derartigen bekannten implantierbaren Defibrillatoren wird nach einer Detektion eines Herzkammerflimmerns ein in dem Defibrillator angeordnetar Kondensator über eine Spannungsquelle auf eine Hochspannung aufgeladen und anschließend mittels am Herzen angeordneten Elektroden über das dazwischen liegende Herzgewebe entladen. Wenn der über das Herz fließende Strom eine ausreichende Energie aufweist, wird dadurch das Herzkammerflimmern beseitigt. Um die zur erfolgreichen Defibrillation ausreichende minimale Energiemenge bestimmen zu können, ist es bekannt, auf künstlichem Wege ein Herzkammerflimmern zu induzieren und anschließend Defibrillationsversuche mit zunehmender Energie durchzuführen, bis das Herzkammerflimmern erfolgreich beendet wird. Das Herzkammerflimmern kann beispielsweise dadurch induziert werden, daß der Patient von außen mit einem 50 Hz Wechselstrom aus einem externen Generator beaufschlagt wird. Ferner ist es möglich, das Herzkammerflimmern durch eine geeignete Stimulationsimpulsfolge eines Herzschrittmachers zu erzeugen, der entweder separat zu dem implantierbaren Defibrillator oder mit diesem zusammen in einem gemeinsamen Gehäuse angeordnet sein kann.

Der Erfindung liegt die Aufgabe zugrunde, einen implantierbaren Defibrillator anzugeben, mit dessen Hilfe auf einfachste Weise ein Herzkammerflimmern induziert werden kann.

Gemäß der Erfindung wird die Aufgabe dadurch gelöst, daß bei dem Defibrillator der eingangs angegebenen Art zur Erzeugung des Herzkammerflimmerns der Kondensator bis zum Erreichen einer vorgegebenen Ladespannung, die wesentlich unter der für eine Defibrillierung vorgesehenen Spannung liegt, an eine Spannungsquelle geschaltet wird und daß der Kondensator nach Erreichen der vorgegebenen Ladespannung während kurzer, im Rahmen einer Sequenz intervallweise aufeinanderfolgender Zeitabschnitte von der Spannungsquelle getrennt und mit den Elektroden verbunden wird. Mit dem implantierbaren Defibrillator läßt sich also ein Herzkammerflimmern erzeugen, ohne daß hierzu ein eigens dafür vorgesehener Impulsgenerator oder besondere Elektroden erforderlich sind. Statt dessen erzeugt der erfindungsgemäße Defibrillator mit seinen bereits vorhandenen und zur Defibrillation vorgesehenen Mitteln die zur Induzierung des Herzkammerflimmerns erforderliche Impulssequenz. Da die entsprechenden Impulse über die in der Regel großflächigen Elektroden direkt an das Herz abgegeben werden, kann die Impulsenergie auf einen relativ geringen Wert begrenzt werden, so daß einerseits eine Schädigung des Herzgewebes vermieden, andererseits aber eine sichere Induzierung des Herzkammerflimmerns erreicht wird. Die Spannung der Impulse kann beispielsweise 23 Volt betragen und liegt damit wesentlich unter der für eine Defibrillierung vorgesehenen Spannung. Die der Impulsdauer entsprechende Dauer der Zeitabschnitte liegt vorzugsweise in der Größenordnung von 8 ms, während die der Impulspause ensprechende Intervalldauer vorzugsweise in der Größenordnung von 25 ms liegt.

Die Spannungsamplitude der Impulse wird in vorteilhafter Weise dadurch eingestellt, daß zur Feststellung des Erreichens der vorgegebenen Ladespannung die Spannung über dem Kondensator gemessen wird.

Alternativ hierzu ist entsprechend einer bevorzugten Ausführung des erfindungsgemäßen Defibrillators vorgesehen, daß der Kondensator von der Spannungsquelle mit einem vorgegebenen Ladestrom aufgeladen wird, und daß das Erreichen der vorgegebenen Ladespannung durch Ablauf einer vorgegebenen Zeit nach dem Beginn des Aufladevorganges definiert wird.

Bei einer bevorzugten Ausführung des erfindungsgemäßen Defibrillators ist der Ladestrom für den Kondensator zumindest während der Sequenzdauer veränderbar, so daß Impulssequenzen mit einer stetigen Zunahme oder Abnahme der Impulshöhe der einzelnen Impulse erzeugt werden können.

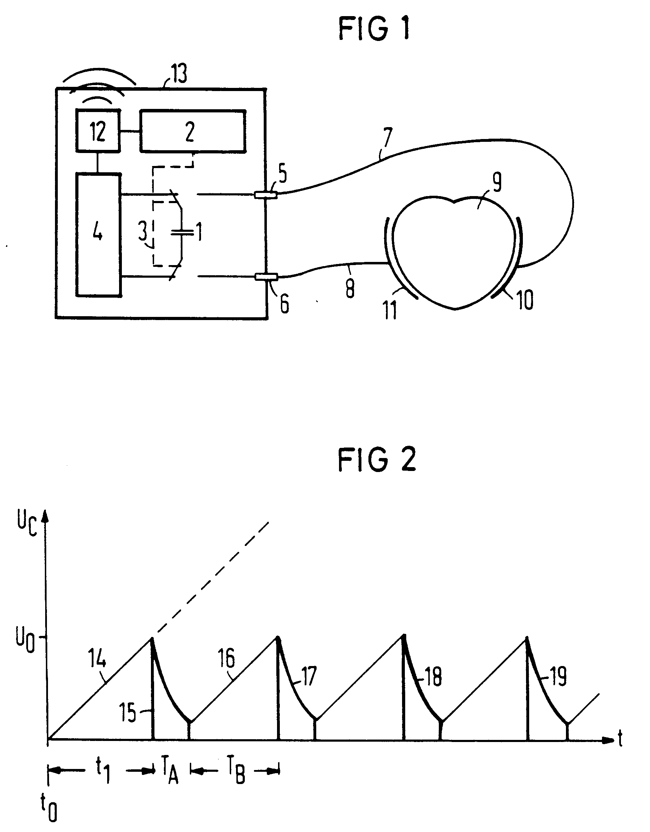

Zur Erläuterung der Erfindung wird im folgenden auf die Figuren der Zeichnung Bezug genommen; im einzelnen zeigen FIG 1 ein Blockschaltbild eines bevorzugten Ausführungsbeispiels des erfindungsgemäßen Defibrillators und FIG 2 eine Beispiel für eine von dem erfindungsgemäßen Defibrillator erzeugte Impulssequenz zur Induzierung eines Herzkammerflimmerns.

FIG 1 zeigt ein Ausführungsbeispiel für einen implantierbaren Defibrillator, bei dem ein Kondensator 1 über eine von einer Steuereinrichtung 2 steuerbare Schalteranordnung 3 entweder an eine Spannungsquelle 4 oder an Elektrodenanschlüsse 5 und 6 schaltbar ist, an denen über jeweils eine Elektrodenleitung 7 bzw. 8 zwei direkt an einem Herzen 9 angeordnete Elektroden 10 und 11 angeschlossen sind. Der Kondensator 1, die Steuereinrichtung 2, die Schalteranordnung 3, die Spannungsquelle 4 sowie eine mit der Steuereinrichtung 2 und der Spannungsquelle 4 verbundene Telemetrieeinheit 12 zur Kommunikation mit einem äußeren, hier nicht dargestellten Programmiergerät, sind in einem implantierbaren Kapselgehäuse 13 angeordnet, wobei die Elektrodenanschlüsse 5 und 6 in Form von Durchführungen durch die Wand des Kapselgehäuses 13 ausgebildet sind.

Zur Defibrillation des Herzens 9 wird der Kondensator 1 über die steuerbare Schalteranordnung 3 mit der Spannungsquelle 4 verbunden und auf eine Hochspannung aufgeladen. Anschließend wird der Kondensator 1 über die steuerbare Schalteranordnung 3 mit den Elektrodenanschlüssen 5 und 6 verbunden, wobei sich der Kondensator 1 über die Elektroden 10 und 11 und das dazwischen liegende Herzgewebe entlädt und dabei im Falle einer ausreichenden Energiemenge des Entladestromes eine Defibrillation bewirkt. Es ist daher im Zusammenhang mit der Implantation des Defibrillators erforderlich, die geringste, zur erfolgreichen Defibrillation ausreichende Energiemenge festzustellen. Dazu wird in dem Herzen 9 ein Herzkammerflimmern induziert woraufhin anschließend mehrere Defibrillationsversuche jedesmal mit einer höheren Energiemenge durchgeführt werden, bis eine erfolgreiche Defibrillation des Herzens erfolgt ist. Die Induzierung des Herzkammerflimmerns mit Hilfe des erfindungsgemäßen Defibrillators wird im folgenden an Hand von FIG 2 näher erläutert.

Zu einem Zeitpunkt tₒ wird der Kondensator 1 über die steuerbare Schalteranordnung 3 mit der Spannungsquelle 4 verbunden und entsprechend dem mit 14 bezeichneten Spannungsverlauf aufgeladen. Die Spannung über dem Kondensator 1, also die Ladespannung ist mit U_{C} bezeichnet. Bei einer vorgegebenen Ladespannung U₀, die entweder durch Messen der Spannung U_{C} über dem Kondensator oder bei einem vorgegebenen Ladestrom durch den Ablauf einer vorgegebenen Zeit t₁ nach dem Beginn t₀ des Aufladevorgangs ermittelt werden kann, wird der Kondensator 1 durch die steuerbare Schalteranordnung 3 von der Spannungsquelle 4 getrennt und für einen kurzen Zeitabschnitt T_{A} mit den Elektrodenanschlüssen 5 und 6 verbunden. Dabei entlädt sich der Kondensator 1 unter Abgabe des mit 15 bezeichneten Impulses über das zwischen den Elektroden 10 und 11 liegende Herzgewebe. Am Ende des Zeitabschnittes T_{A} wird der Kondensator 1 wieder an die Spannungsquelle 4 geschaltet und während einer Intervalldauer T_{B} entsprechend dem mit 16 bezeichneten Spannungsverlauf aufgeladen. Gegen Ende der Intervalldauer T_{B} wird der Kondensator 1 wiederum für einen Zeitabschnitt T_{A} über das Herzgewebe 9 entladen. Dieser Vorgang wiederholt sich über eine Sequenz von mehreren Impulsen 15,17,18 und 19, wodurch ein Herzkammerflimmern induziert wird. Durch Änderung des Ladestromes, was in FIG 2 einer Änderung der Steigung der Spannungsverläufe 14 und 16 entspricht, können bei unverändeter Intervalldauer T_{B} Sequenzen mit stetig ansteigender oder abfallender Impulshöhe erzeugt werden.

### Bezugszeichenliste

- 1: Kondensator
- 2: Steuereinrichtung
- 3: steuerbare Schalteranordnung
- 4: Spannungsquelle
- 5,6: Elektrodenanschlüsse
- 7,8: Elektrodenleitungen
- 9: Herz
- 10,11: Elektroden
- 12: Telemetrieeinrichtung
- 13: Kapselgehäuse
- 14,16: Spannungsverläufe an 1
- 15,17,18,19: Impulse
- t: Zeit
- t₀: Zeitpunkt
- t₁: Zeitablauf
- T_{A}: Zeitabschnitt (Impulsdauer)
- T_{B}: Intervalldauer (Impulspause)
- U₀: vorgegebene Ladespannung von 1
- U_{C}: Spannung über 1

## Patentansprüche

1. Implantierbarer Defibrillator mit einer steuerbaren Schalteranordnung (3), mit Elektrodenanschlüsse (5,6), mit einem Kondensator (1), der über die steuerbare Schalteranordnung (3) wechselweise mit einer Spannungsquelle (4) oder die Elektrodenanschlüsse (5,6) verbindbar ist, und mit einer die Schalteranordnung steuernden Steuereinrichtung (2), **dadurch gekennzeichnet**, dass die Schalteranordnung (3) von der Steuereinrichtung (2) so gesteuert ist, dass zur Erzeugung eines Herzkammerflimmerns der Kondensator (1) bis zum Erreichen einer vorgegebenen Ladespannung U₀, die wesentlich unter der für eine Defibrillierung vorgesehenen spannung liegt, während einer Intervalldauer T_{B} an die Spannungsquelle (4) geschaltet wird und dass der Kondensator (1) nach Erreichen der vorgegebenen Ladespannung U₀ während kurzer, im Rahmen einer Sequenz intervallweise aufeinanderfolgender Zeitabschnitte T_{A} von der Spannungsquelle (4) getrennt und mit den Elektrodenanschlüssen (5,6) verbunden wird.

2. Implantierbarer Defibrillator nach Anspruch 1, **dadurch gekennzeichnet**, daß die Dauer der Zeitabschnitte T_{A} im wesentlichen 8 ms ist.

3. Implantierbarer Defibrillator nach Anspruch 1 oder 2, **dadurch gekennzeichnet**, daß die Intervalldauer T_{B} im wesentlichen 25 ms ist.

4. Implantierbarer Defibrillator nach einem der vorangehenden Ansprüche, **dadurch gekennzeichnet,** daß zur Feststellung des Erreichens der vorgegebenen Ladespannung U₀ die Spannung U_{C} über dem Kondensator (1) gemessen wird.

5. Implatierbarer Defibrillator nach einem der Ansprüche 1 bis 3, **dadurch gekennzeichnet**, daß der Kondensator (1) von der Spannungsquelle (4) mit einem vorgegebenen Ladestrom aufgeladen wird und daß das Erreichen der vorgegebenen Ladespannung U₀ durch Ablauf einer vorgegebenen Zeit t₁ nach den Beginn t₀ des Aufladevorganges definiert wird.

6. Implantierbarer Defibrillator nach Anspruch 5, **dadurch gekennzeichnet**, daß der Ladestrom für den Kondensator 1 zumindest während der Sequenzdauer veränderbar ist.

## Claims

1. Implantable defibrillator having a controllable switch arrangement (3), having electrode terminals (5, 6), having a capacitor (1) which can be connected by way of the controllable switch arrangement (3) alternately to a voltage source (4) or the electrode terminals (5, 6), and having a control device (2) controlling the switch arrangement, characterized in that the switch arrangement (3) is controlled by the control device (2) in such a way that to produce a heart ventricular fibrillation the capacitor (1) is connected to the voltage source (4) during an interval T_{B} until a given charging voltage U_{O} is reached, which lies substantially below the voltage provided for a defibrillation, and in that the capacitor (1), after reaching the given charging voltage U_{O}, during short time segments T_{A} succeeding each other at intervals within the scope of a sequence, is separated from the voltage source (4) and is connected to the electrode terminals (5, 6).

2. Implantable defibrillator according to claim 1, characterized in that the duration of the time segments T_{A} is substantially 8 ms.

3. Implantable defibrillator according to claim 1 or 2, characterized in that the interval T_{B} is substantially 25 ms.

4. Implantable defibrillator according to one of the preceding claims, characterized in that to ascertain the reaching of the given charging voltage U_{O} the voltage U_{C} over the capacitor (1) is measured.

5. Implantable defibrillator according to one of claims 1 to 3, characterized in that the capacitor (1) is charged by the voltage source (4) with a given charging current and in that the reaching of the given charging voltage U_{O} is defined by the passing of a given time t₁ after the beginning t_{O} of the charging procedure.

6. Implantable defibrillator according to claim 5, characterized in that the charging current for the capacitor 1 can be changed at least during the sequence duration.

## Revendications

1. Défibrillateur implantable comportant un dispositif (3) d'interrupteur commandable, des bornes (5,6) d'électrode, un condensateur (1), qui peut être relié par l'intermédiaire du dispositif (3) d'interrupteur commandable en alternance à une source (4) de tension ou aux bornes (5,6) d'électrode, et comportant un dispositif (2) de commande commandant le dispositif d'interrupteur, caractérisé en ce que le dispositif (3) d'interrupteur est commandé par le dispositif (2) de commande, de telle sorte que le condensateur (1), soit branché, pendant une durée T_{B} d'intervalle à la source (4) de tension, pour produire une fibrillation ventriculaire, jusqu'à ce qu'une tension U₀ de charge prescrite soit atteinte, laquelle est sensiblement inférieure à la tension prévue pour une défibrillation et que le condensateur (1) soit séparé, après que la tension U₀ de charge prescrite soit atteinte, pendant de courts laps T_{A} de temps se succédant à intervalle dans le cadre d'une séquence, de la source (4) de tension et soit relié aux bornes (5,6) d'électrode.

2. Défibrillateur implantable suivant la revendication 1, caractérisé en ce que la durée des laps T_{A} de temps est sensiblement de 8 ms.

3. Défibrillateur implantable suivant la revendication 1 ou 2, caractérisé en ce que la durée T_{B} d'intervalle est sensiblement de 25 ms.

4. Défibrillateur implantable suivant l'une des revendications précédentes, caractérisé en ce que la tension U_{C} est mesurée aux bornes du condensateur (1) pour fixer l'atteinte de la tension U₀ de charge prescrite.

5. Défibrillateur implantable suivant l'une des revendications 1 à 3, caractérisé en ce que le condensateur (1) est chargé par la source (4) de tension à un courant de charge prescrit et l'atteinte de la tension U₀ de charge prescrite est définie par l'écoulement d'un temps prescrit t₁ après le début t₀ de l'opération de charge.

6. Défibrillateur implantable suivant la revendication 5, caractérisé en ce que le courant de charge pour le condensateur 1 peut être modifié au moins pendant la durée de la séquence.
